# EUROPEAN PATENT SPECIFICATION

(11) **EP 4 176 429 B1**
(45) Date of publication and mention of the grant of the patent: **28.10.2026**
(21) Application number: 21737389.3
(22) Date of filing: 25.06.2021
(51) Int. Cl.: G09B 19/24, G09B 23/28

(54) **APPARATUS AND MECHANISM FOR SIMULATING DENTAL PROCEDURES AND METHODS**
VORRICHTUNG UND MECHANISMUS ZUR SIMULATION VON ZAHNÄRZTLICHEN EINGRIFFEN UND VERFAHREN
APPAREILS ET MÉCANISMES POUR LA SIMULATION D'INTERVENTIONS ET DE PROCÉDÉS DENTAIRES

(30) Priority: 02.07.2020 DK PA202070452
(43) Date of publication of application: 10.05.2023
(73) Proprietor: Simtolife B.V., 1068 KK Amsterdam (NL)
(72) Inventor: BODE, Dyon, 1260 Copenhagen K (DK)
(74) Representative: Nordic Patent Service A/S
(86) International application number: PCT/EP2021/067463
(87) International publication number: WO 2022/002778

(56) References cited:
- EP-A1- 2 988 288
- JP-A- 2015 171 447
- US-A- 4 968 256
- HADAVAND M ET AL: "Design of a force-reflective master robot for haptic telesurgery applications: RoboMaster1", ENGINEERING IN MEDICINE AND BIOLOGY SOCIETY,EMBC, 2011 ANNUAL INTERNATIONAL CONFERENCE OF THE IEEE, IEEE, 30 August 2011 (2011-08-30), pages 7037 - 7040, XP032111484, ISBN: 978-1-4244-4121-1, DOI: 10.1109/IEMBS.2011.6091779
- ROSEN J ET AL: "The BlueDRAGON - a system for measuring the kinematics and dynamics of minimally invasive surgical tools in-vivo", PROCEEDINGS / 2002 IEEE INTERNATIONAL CONFERENCE ON ROBOTICS AND AUTOMATION : MAY 11 - 15, 2002, WASHINGTON, D.C, IEEE SERVICE CENTER, PISCATAWAY, NJ, vol. 2, 11 May 2002 (2002-05-11), pages 1876, XP032882995, ISBN: 978-0-7803-7272-6, DOI: 10.1109/ROBOT.2002.1014814
- MADHANI A J ET AL: "The Black Falcon: a teleoperated surgical instrument for minimally invasive surgery", INTELLIGENT ROBOTS AND SYSTEMS, 1998. PROCEEDINGS., 1998 IEEE/RSJ INTE RNATIONALCONFERENCE ON, IEEE, vol. 2, 13 October 1998 (1998-10-13), pages 936 - 944, XP010311544, ISBN: 978-0-7803-4465-5, DOI: 10.1109/IROS.1998.727320

## Description

### TECHNICAL FIELD

The disclosure relates to apparatuses for simulating medical procedures and methods and a mechanism for use in such apparatuses, in particular apparatuses and methods that use virtual, mixed, and/or augmented reality to simulate the activities of a medical practitioner.

### BACKGROUND

Some known virtual medical simulators involve a computer that provides a simulated environment using a display screen to show the simulated environment to the users and a haptic arm to provide haptic force feedback based on events in the simulated environment. Examples of virtual medical simulators are e.g. dental simulators, eye surgery simulators, and arthroscopic surgery simulators.

Dentistry, also known as Dental and Oral Medicine, is a branch of medicine that consists of the study, diagnosis, prevention, and treatment of diseases, disorders, and conditions of the oral cavity, commonly in the dentition but also the oral mucosa, and of adjacent and related structures and tissues, particularly in the maxillofacial (jaw and facial) area. Dentistry encompasses practices related to the oral cavity, as performed by dentists, dental surgeons, oral surgeons, maxillofacial surgeons, dental hygienists, and dental therapists in the form of dental procedures and treatments.

Dental students require facilities for training, and using real patients has obvious drawbacks, for example, the risk of transmission of contagious diseases and possible suffering of patients due to imperfect procedures by inexperienced practitioners.

Dental simulators for simulating dental procedures are known in the art. These simulators are used to train dentistry students thereby reducing the need for training on plastic phantom heads with plastic phantom teeth (which do not provide an accurate simulation, do not allow for objective assessment or tracking of work, and are not environment-friendly) and reducing the need for training on real patients. Known dental simulators comprise a computer that controls the simulation and hosts a virtual environment, a display screen displaying the simulated environment, and one or two handpieces connected to the computer to provide input. The simulated environment comprises the subject, a set of virtual teeth, virtual versions of tools controlled by the handpieces, as well as a virtual version of the handpieces themselves. The tools may be surgical instruments (scalpels, syringes, etc.) or other devices (such as mirrors, guides, or probes). The handpieces are connected to sensors that determine their position and orientation, which is used to control (display) the position of the tools in the virtual environment. Typically, one of the handpieces is mounted on a haptic feedback system through which the computer controls the forces the user feels through the handpiece.

A static U-shaped rail or the like serves as a handrest for the hands/fingers of the dentistry student (user). In real dental procedures or treatments, the dentist will typically rest his fingers on the patient's teeth and jaw, and a simulation with a single fixed U-shaped rail in the known dental simulator is therefore not a realistic simulation.

The visual display screen is disposed between the user's eyes and the handpieces and the rail and presents a virtual environment that shows a virtual set of teeth on a virtual jaw. The known simulator comprises a computer that controls the simulation and hosts a virtual environment, a display screen displaying the simulated environment, and one or two handpieces that are connected to the computer to provide an input/output. The simulated environment comprises the subject, as well as virtual versions of tools controlled by the handpieces. The tools may be surgical instruments (scalpels, syringes, etc.) or other devices (such as mirrors or probes). The handpieces are connected to sensors that determine their position, which is used to control the position of the tools in the virtual environment. One of the handpieces is mounted on a haptic feedback system which allows the computer to control the forces the user feels through the handpieces, making a more realistic simulation possible. Thus, a virtual version of the handpieces is displayed on the display screen, but the user cannot see his own fingers or hands, which is a drawback since it denies the user an important visual input.

The above referred known dental simulators use a haptic arm that is connected to the distal end of the medical (dental) handpiece (the end that is provided with the end effector, that is e.g. shaped like a head in a dental handpiece) via a curved link using revolute joints to allow the handpiece to rotated about three orthogonal axes that substantially coincide at or near the distal end. However, the distal end is the end that is manipulated in the workspace and this known construction has the drawback that the haptic arm and in particular, the link that connects the haptic arm to the handpiece protrudes into the workspace.

EP2988288 discloses a medical procedure simulator according to the preamble of claim 1.

### SUMMARY

It is an object to provide a medical simulator that overcomes or at least reduces at least one of the problems mentioned above.

The foregoing and other objects are achieved by the features of the independent claims. Further implementation forms are apparent from the dependent claims, the description, and the figures.

According to a first aspect, there is provided a medical procedure simulator for simulating a medical treatment or procedure, the medical procedure simulator comprising:
a handpiece with a proximal end and a distal end, the handpiece being configured to be manipulated by a user in a workspace in real space, a haptic arm controlled by a computer that is configured to simulate a medical procedure or treatment though haptic feedback, preferably haptic force feedback, to the handpiece with the haptic arm, a mechanism connected to the proximal end of the handpiece and connected to the haptic arm at a connection position on the haptic arm, the mechanism comprising a first parallel four-bar linkage coupled to a second parallel four-bar linkage, the mechanism being configured to allow the handpiece to rotate about three orthogonal axes that intersect through a common point in the workspace,
the common point being a fixed position relative to the connection position so that the common point moves in unison with the connection position, and
the common point being located at a fixed distance from the connection position.

The mechanism allows the handpiece to be connected at its proximate end while maintaining the axes of rotation of the handpiece at or near the distal end of the handpiece (at the common point) and with the connection position on the haptic arm at a fixed distance from the common point.

The common point preferably corresponds to the tip of a virtual tool that is virtually connected to the distal end of the handpiece. Thus, all rotation is about the tip of the virtual tool.

In case the medical procedure simulator is a dental simulator, the virtual tool will typically be a virtual burr.

The user experiences the forces of the haptic force feedback as acting on the distal end, like they do in a real medical procedure. Thus, the mechanism allows the handpiece to protrude with its distal end into the workspace without any other part that is connected to the haptic arm protruding into the workspace. This facilitates improvement of simulation of treatments or procedures that are performed (deep) in a cavity of the human or animal body, which is the case for many types of medical treatment or procedures. Further, it becomes possible to arrange a phantom body part, e.g. a phantom body cavity in which the handpiece protrudes with its distal end, without any other part that is connected to the haptic force feedback arm risking abutting with the phantom body part (and thus undermining the reality of the simulation). Another example of a phantom part could be a phantom neighboring tooth to the virtual tooth, which can act as a realistic finger rest for the user. Accordingly, the simulation is rendered more realistic, and simulation is possible in a recess or cavity formed by phantom body parts.

According to a possible implementation form of the first aspect, the common point is located at a fixed distance (D) from the connection position.

According to a possible implementation form of the first aspect, the handpiece represents a real medical handpiece with a real proximal end and a real distal end, the real distal end being shaped like a real distal and that is configured being provided with a real end effector. In the medical simulator, the end effector is a virtual end effector.

According to a possible implementation form of the first aspect, the distal end represents the distal end of a real medical handpiece.

According to a possible implementation form of the first aspect, the handpiece is shaped and sized like a real medical handpiece.

According to a possible implementation form of the first aspect, the connection position is at the free end of the haptic arm.

□aspect, the computer is configured to provide a virtual environment including a virtual end effector that simulates the real end effector.

According to a possible implementation form of the first aspect, the distal end is the end where the simulated forces from a virtual end effector are perceived to act on the handpiece.

According to a possible implementation form of the first aspect, the handpiece is shaped and sized like a dental drill and wherein the distal end is shaped like a dental drill head and wherein the medical procedure or treatment is preferably a dental procedure or treatment.

According to a possible implementation form of the first aspect, the computer is configured to simulate the medical procedure or treatment through force feedback in response to manipulation of the handpiece by the user in the workspace.

According to a possible implementation form of the first aspect, the common point substantially coincides with the distal end.

According to a possible implementation form of the first aspect, the mechanism provides a remote common point of rotation for a plurality of axes for the handpiece.

According to a possible implementation form of the first aspect, the first parallel four-bar linkage is operatively coupled with the second parallel four-bar linkage to allow rotation of the handpiece about a second axis coinciding with the common point.

According to a possible implementation form of the first aspect the second parallel four-bar linkage is connected to the haptic arm by a second revolute joint, the second revolute joint allowing rotation about a third axis parallel with the extent of the haptic arm, and the third axis preferably coinciding with the common point.

According to a possible implementation form of the first aspect, the mechanism comprises a first arm that is connected to the proximal end by a revolute joint that allows the handpiece to rotate about a first axis that coincides with the longitudinal axis of the handpiece, the first arm preferably being part of a first pair of parallel arms of the first parallel four-bar linkage.

According to a possible implementation form of the first aspect, the haptic arm comprises linkage comprising a main link that is operably coupled to a reference by actuators, the main link comprising a three-dimensional force sensor for sensing forces applied by the user to the handpiece in three dimensions, the three-dimensional force sensor being disposed between the connection position and any position at which the actuators connect to the main link, and the three-dimensional force sensor preferably being an integral part of the main link.

According to a possible implementation form of the first aspect, the first revolute joint is provided with a rotary position sensor for sensing the rotary position of the handpiece relative to the first arm.

According to a possible implementation form of the first aspect, an inertial measurement unit is arranged inside the handpiece.

According to a possible implementation form of the first aspect, the medical procedure simulator comprises a phantom upper jaw and a phantom lower jaw, preferably as a part of a phantom head, the phantom lower jaw being arranged at a jaw angle to the phantom upper jaw, the jaw angle not exceeding 55°, preferably not exceeding 50° and even more preferably not exceeding 45°, and the connection position being located, inferior, preferably inferomedial to the lower jaw.

According to a possible implementation form of the first aspect, the lower jaw is arranged pivotable relative to the upper jaw between a closed position and an open position, the maximum angle between the upper jaw and the lower jaw being limited not to exceed 55°, preferably not to exceed 50° and even more preferably not to exceed 45°.

According to a possible implementation form of the first aspect, the default orientation of the phantom head corresponds to a position of a patient in a fully reclined dental chair, i.e. with the face of the patient being directed substantially upwards.

According to a possible implementation form of the first aspect, the rotation axis of the handpiece is powered and the torque applied to the handpiece is controlled for providing haptic feedback on the orientation of the handpiece.

According to a possible implementation form of the first aspect, a force sensor is associated with the handpiece, preferably a torque sensor and more preferably a three-dimensional torque sensor.

According to a possible implementation form of the first aspect the computer is configured to simulate the medical procedure or treatment through haptic feedback, preferably haptic force control feedback, with the haptic arm and through visual feedback with a display screen.

According to a possible implementation form of the first aspect, the connection position on the haptic arm is at or near an extremity of the haptic arm.

According to a possible implementation form of the first aspect, the medical procedure simulator comprises a reference, wherein the haptic arm linkage provides at least six independent degrees of freedom at the connection position relative to the reference.

According to a possible implementation form of the first aspect, the handpiece comprises a handle part that extends from the proximal end towards the distal end and is connected to the mechanism at its proximal end.

According to a possible implementation form of the first aspect, the haptic arm has a controlled end, and wherein the connection position is at or near the controlled end.

According to a possible implementation form of the first aspect the medical procedure simulator comprises a display screen, and wherein the computer is configured to display a virtual environment on the display screen, the virtual environment comprising at least a virtual handpiece that is provided with a virtual end effector, the computer being configured to co-locate the virtual handpiece and the virtual end effector with the handpiece).

According to a second aspect, there is provided a mechanism for connecting a handpiece of a medical simulator to a haptic arm of a medical simulator according to the first aspect or any possible implementations thereof, the mechanism comprising: a first arm for connecting to the handpiece via a first revolute joint, the first revolute joint allowing the handpiece to rotate about a first axis that coincides with a longitudinal axis of the handpiece, the first arm being part of a first parallel four-bar linkage, the first parallel four-bar linkage being operably connected to a second four-bar linkage to allow the handpiece to rotate about a second axis, the second parallel four-bar linkage comprising a second arm for connecting to the haptic arm via a second revolute joint, the second revolute joint allowing the handpiece to rotate about a third axis that is parallel with the haptic arm.

According to a possible implementation of the second aspect, the mechanism comprises a real handpiece with a real proximal end and a real distal end, the real distal end shaped and sized like a distal end of a real medical handpiece configured for being provided with a real end effector to perform the medical treatment or procedure.

According to a possible implementation of the second aspect, the distal end represents the real distal end of the real medical handpiece.

According to a possible implementation of the second aspect, the handpiece is shaped and sized like the real medical handpiece.

According to a possible implementation of the second aspect, the distal end is the end where the simulated forces from a virtual end effector act on the handpiece.

According to a possible implementation of the second aspect, the handpiece is shaped and sized like a dental drill and wherein the distal end is shaped like a dental drill head, and wherein the medical procedure or treatment is preferably a dental procedure or treatment.

According to a possible implementation of the second aspect, the second axis is at an angle, preferably a right angle, to the third axis.

According to a possible implementation of the second aspect, the first, second, and third axis substantially coincide at a common point at or near the distal end.

According to a possible implementation of the second aspect, the common point moves in unison with the second revolute joint.

According to a possible implementation of the second aspect, the first, second, and third axes intersect at a position in space that is fixed relative to the second revolute joint, and at a fixed distance to the second revolute joint.

According to a possible implementation of the second aspect, the first parallel four-bar linkage comprises a first pair of parallel links and a first pair of parallel arms,
wherein the second parallel four-bar linkage comprises a second pair of parallel links and a second pair of parallel arms,
wherein the first pair of parallel arms is formed by the first arm and by an arm formed by an extension of one link of the second pair of parallel links, and
wherein the second pair of parallel arms is formed by the second arm and by an arm formed by an extension of one link of the first pair of parallel links.

According to a possible implementation of the second aspect one link of the first pair of links and one link of the second pair of links is formed by two parallel members that are spaced in the direction of the second axis, the two parallel members preferably being interconnected by bracing.

According to a possible implementation of the second aspect, the links of the first pair of links are kinked.

According to a possible implementation of the second aspect, the mechanism is equipoised by one or more resilient members operably coupled to the mechanism, the resilient members preferably being long elastic cords or cables.

According to a possible implementation of the second aspect, the first parallel four-bar linkage and/or the second parallel four-bar linkage are enveloped by a covering.

According to a possible implementation of the second aspect, the links of the first parallel four-bar linkage and the links of the second parallel four-bar linkage move in parallel planes,
the first parallel four-bar linkage and the second, parallel four-bar linkage are planar four-bar linkages.

These and other aspects will be apparent from the embodiment(s) described below.

### BRIEF DESCRIPTION OF THE DRAWINGS

In the following detailed portion of the present disclosure, the aspects, embodiments, and implementations will be explained in more detail with reference to the example embodiments shown in the drawings, in which:
Fig. 1 is an elevated view of a medical procedure simulator according to an embodiment,
Fig. 2 is a side view of the medical procedure simulator according Fig. 1,
Fig. 3 is a frontal view of a display housing of the dental procedure simulator of Fig. 1,
Fig. 4. is a sectional view through the display housing of

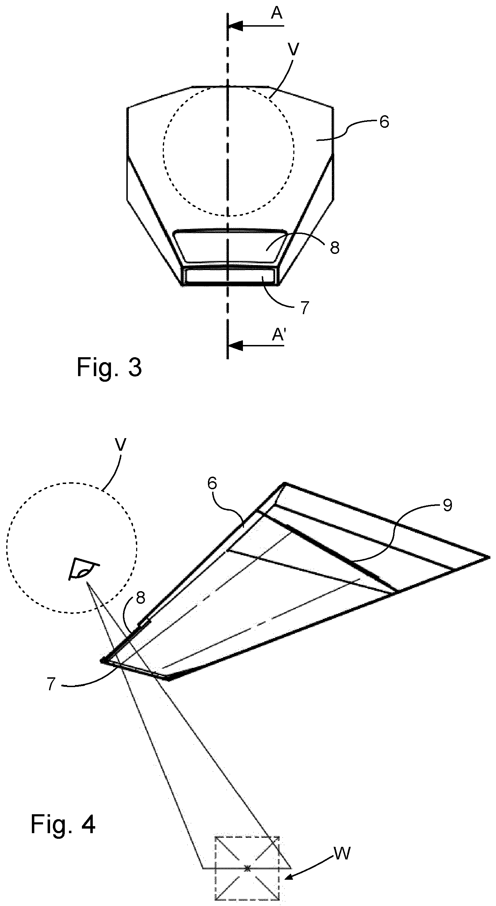

Fig. 3, also illustrating a workspace, the eye of a user, and a vision space,

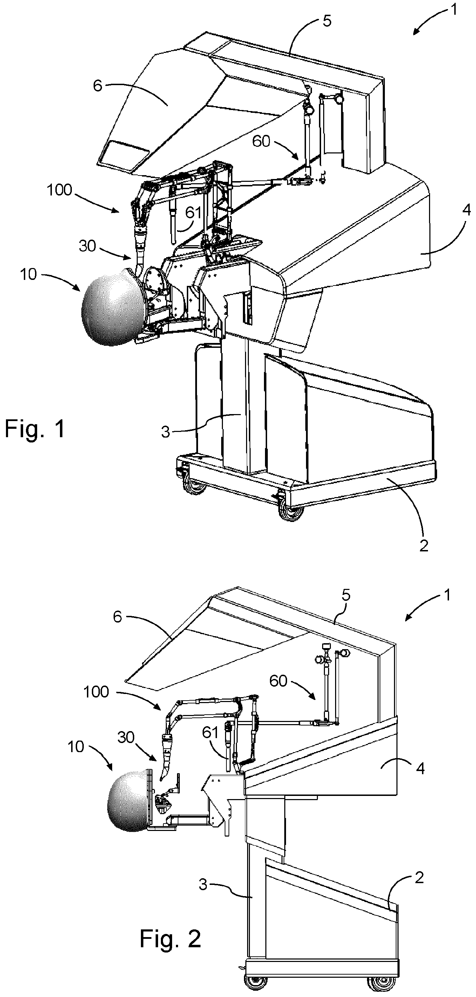

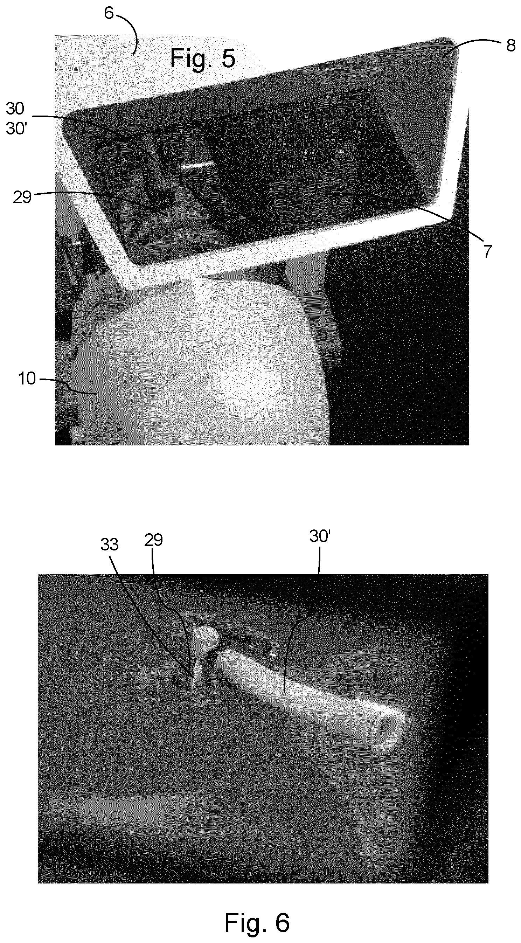

Fig. 5 is an elevated view from a user perspective through a semi-transparent mirror in the display housing to a workspace of the dental procedure simulator of Fig. 1, also showing a virtual environment through reflection from the semi-transparent mirror,
Fig. 6 is an image of a mixed reality virtual environment displayed by the dental procedure simulator of Fig. 1,

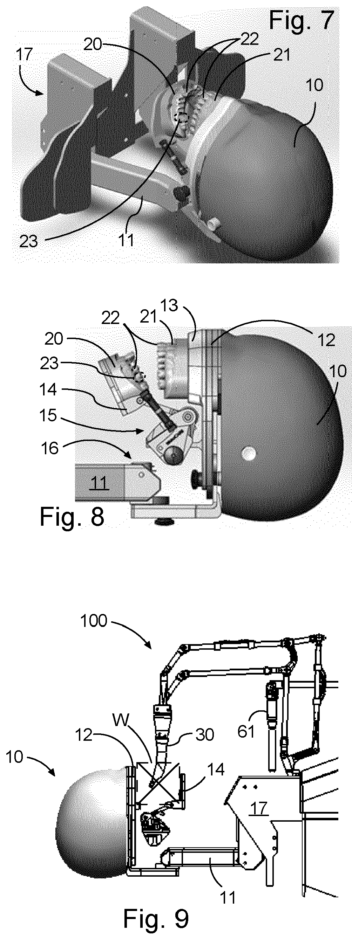

Fig. 7 is an elevated view of a phantom head and its mount system used in the dental procedure simulator of Fig. 1, including a lower specific phantom jaw and a specific phantom upper jaw,
Fig. 8 is a side view of the phantom head of Fig. 7,
Fig. 9 is a side view of the phantom head showing the workspace between the jaws of the phantom head and showing a handpiece and a mechanism connected to a haptic arm of the medical procedure simulator of Fig. 1,

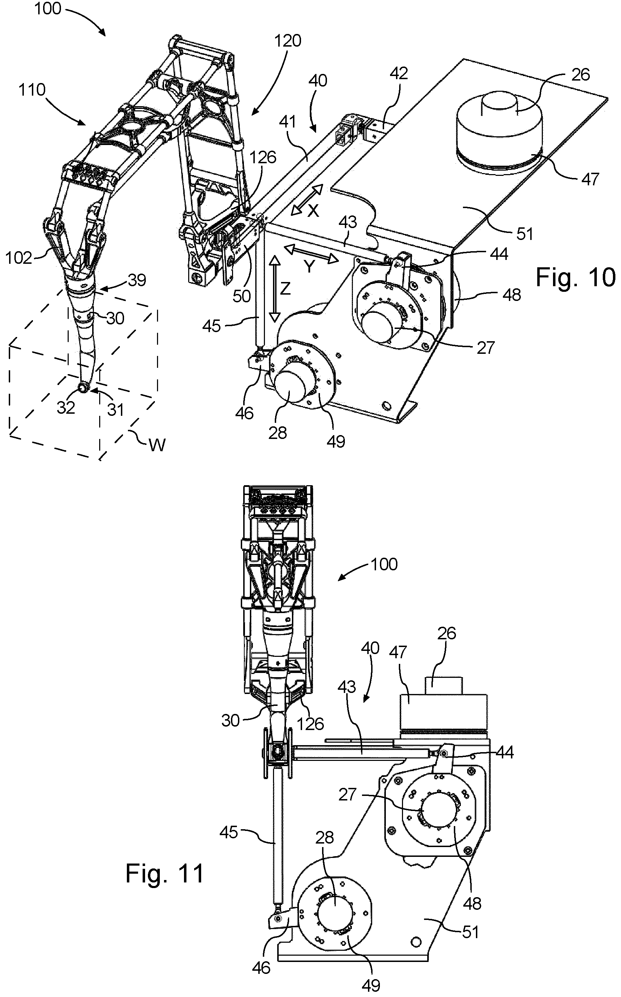

Fig. 10 is an elevated view of an embodiment of a linkage with its drive system and a handpiece that is connected to the linkage, as used in the medical procedure simulator of Fig. 1
Fig. 11 is a front view of the linkage with its drive system and handpiece connected to the linkage of Fig. 10,

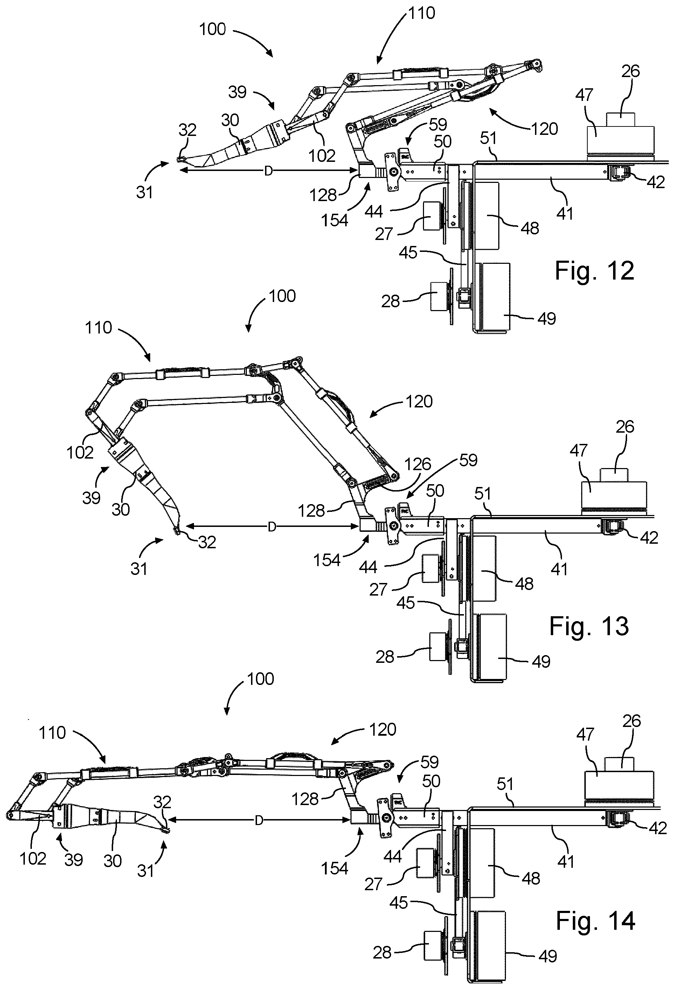

Figs. 12 to 14 are side views of the linkage with its drive system and a handpiece that is connected to the linkage of Fig. 10 by a mechanism, with the handpiece in different orientations,

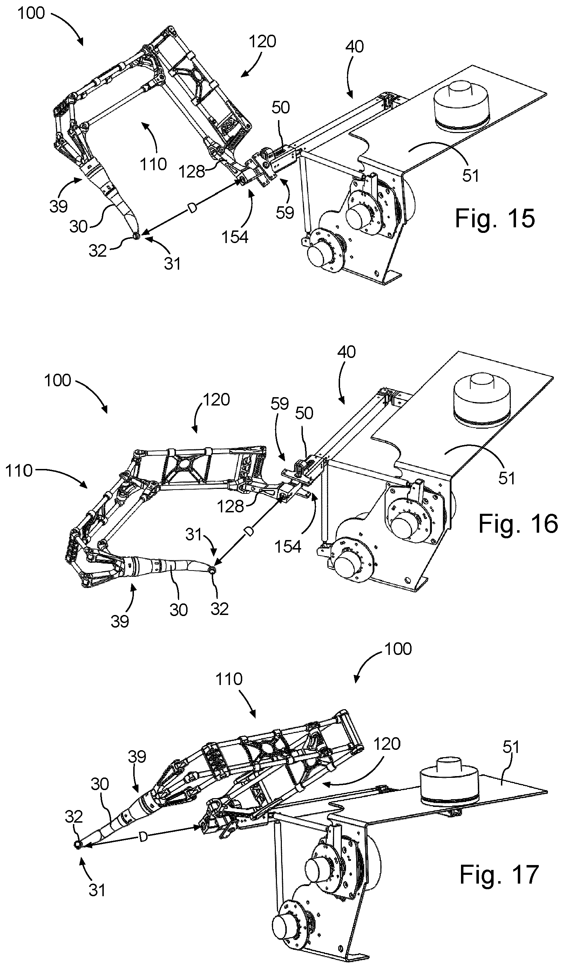

Figs. 15 to 17 are elevated views of the linkage with its drive system and a handpiece that is connected to the linkage of Fig. 10 by a mechanism, with the handpiece, and in different orientations,

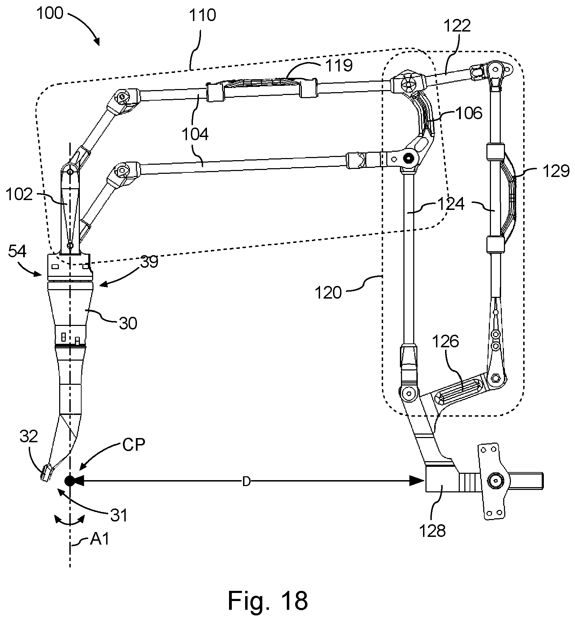

Fig. 18 is a side view of the mechanism and the handpiece according to an embodiment,

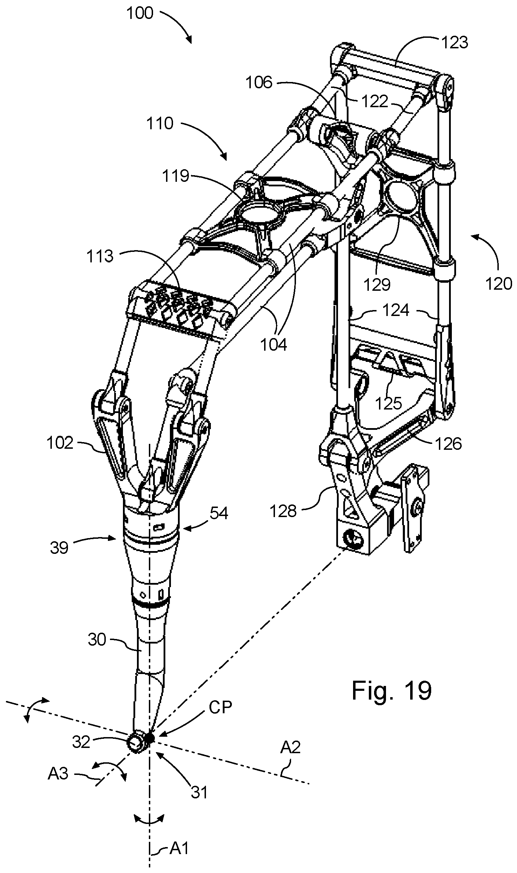

Fig. 19 is an elevated view of the mechanism and handpiece of Fig. 18,

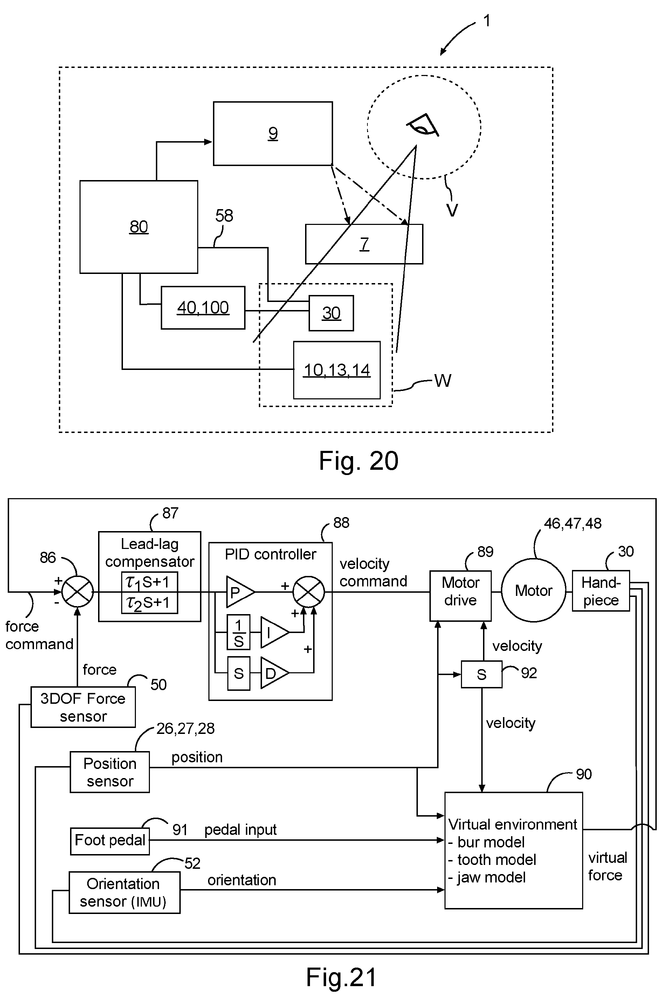

Fig. 20 is a schematic representation of the dental procedure simulator of Fig. 1 showing also an eye of a user and a vision space, and
Fig. 21 is a schematic representation of an embodiment of a control system that can be used in the dental procedure simulator.

### DETAILED DESCRIPTION

Referring to the drawings, and particularly to Figs. 1 to 9 and 20 which show an embodiment of a medical procedure simulator 1, in particular, a dental procedure simulator 1 for simulating dental procedures and treatments. The medical procedure simulator 1, is intended to be used to train the skills and competences of medical professionals or students. In case of a dental procedure simulator 1, the dental procedure simulator is intended to be used to train the skills and competences of dentists, dental surgeons, oral surgeons, maxillofacial surgeons, dental hygienists, and dental therapists. The users that undergo training with the dental procedure simulator can be students or professionals. The dental procedure simulator 1 generally includes a first handpiece 30, in this embodiment a first handpiece 30 that represents a dental drill handle, a base 2, in this embodiment a base with a lower housing that houses a computer 80, a post 3, in this embodiment a height-adjustable column, a main housing 4 that houses a linkage 40 to which the first handpiece 30 is connected, a phantom head 10, and a support arm 5 supporting a display housing 6. In an embodiment, the first handpiece 30 is shaped and sized like a real dental handpiece. The first handpiece 30 has a proximal end 39 and a distal end 31 and the first handpiece 30 comprises a handle part that extends from the proximal end 39 towards the distal end 31.

The arm 5 also carries a mirror tool 60 which comprises a linkage forming a passive arm and a second handpiece 61 attached to the free end of the passive arm simulating a dental mirror.

The base 2 is in an embodiment a wheeled base for allowing the medical procedure simulator 1 to be easily rolled by a user to another position. The post 3 extends from the base 2 to the main housing 4 and supports the main housing 4.

A phantom head 10 is suspended from the front side of the main housing 4 and the display housing 6 is suspended from the main housing 4 by a support arm 5.

The medical procedure simulator 1 comprises a lower housing in which a computer 80 is arranged, together with a power supply for the computer 80 and the other electrical components of the medical procedure simulator 1. In an embodiment, the medical procedure simulator 1 comprises more than one computer.

The computer 80 has a memory and a processor. The processor is arranged to execute software stored on the memory, in particular software configured to simulate a medical procedure or treatment.

The computer 80 is connected to a display screen 9, a model in the workspace, a linkage 40 mounted to the main housing 4 and via the linkage 40 an a mechanism 40 to the first handpiece 30 that is also arranged in a workspace W (or at least the distal end 31 of the handpiece 30 protrudes into the workspace W). The linkage 40 (described in detail below with reference to Figs. 10 and 11) is mechanically connected to the first handpiece 30 via a mechanism 100. The workspace W is a three-dimensional space in the real world and the tip (distal end 31) of the first handpiece 30 can be manipulated by a user within the workspace space W without experiencing constraints from the linkage 40 (constraints caused by the range in the orthogonal directions in real space of the extremity of the linkage to which the first handpiece 30 is connected ).

The model represents part of the subject (for example a phantom upper jaw 13 and lower jaw 14 with or without a set of phantom teeth and with or without a phantom head 10) and provides the necessary mechanical environment for the medical procedure or treatment to take place. For example, the surgeon/dentist can rest his hands on the phantom jaws 13,14, phantom teeth 22 and/or phantom head 10 during the procedure.

The velocity of the handpiece 30 is adjusted in response to the forces the user applies to the first handpiece 30 and the interaction of a virtual drill or dental handpiece 30' with a virtual tooth of a virtual model of a jaw with one or more virtual teeth 29. The virtual environment includes algorithms for determining how the velocity of the virtual dental handpiece 30' should change in response to the sum of the x,y,z forces applied by a user on the first handpiece 30 (from 3DoF sensor 50, see Figs. 10 and 11) and any reaction forces from virtual contact of the virtual drill or dental handpiece 30' with a virtual tooth. The virtual environment uses for some aspects Newtonian physics (i.e. Force = spring constant × deflection) to model the reaction forces between the virtual drill 30' and the virtual tooth, whilst changes in the velocity of the first handpiece 30 are determined using a standard PID control loop. The virtual tooth is assigned a hardness and rigidity. The rigidity correlates to the spring constant a tooth provides when contacted and the hardness correlates to how much work a virtual drill must do in order to drill away a volume of the virtual tooth. The position of the real drill (first handpiece) 30 is used to determine whether there is contact with the virtual tooth.

Once the virtual environment calculates the virtual force acting at the virtual drill 30', it commands this force to the standard PID control loop that controls the velocity of the actuators (described in detail further below) in the system to change the real world velocity of the first handpiece 30. The user senses the movement of the first handpiece 30. While the velocity of the first handpiece 30 is controlled by the dental procedure simulator 1, the orientation of the first handpiece 30 is controlled by the user (in an embodiment (not shown), the orientation of the handpiece 30 is powered along 3 axes by motors to obtain a 6-DoF active version of the simulator, in this embodiment, the torque applied to the handpiece is also measured in 3 dimensions). The system measures the orientation of the first handpiece 30 as controlled by a user, and in response updates the orientation of the virtual drill 30' in the virtual environment.

The movably suspended phantom head 10 is used to adjust the orientation of the virtual environment shown on display 9. The orientation of the phantom head 10 can be manually adjusted and orientation of the virtual model is adjusted accordingly, using sensors coupled to the computer 80 that measure the rotation of the phantom head 10. Thus, the phantom head 10 and virtual phantom head/ or only jaws are co-located and linked. When the user turns the real phantom head 10 the virtual head rotates in the scene. The phantom head 10 is an intuitive control for the virtual model orientation.

The computer 80 provides an interface to the user for selecting different virtual environments procedures and treatments to be simulated and running various training software applications. The training applications monitor the interaction of a user with the virtual environment and first handpiece 30 and measure various criteria to evaluate the performance of a user.

Referring now in particular to Figs. 3 to 6 the visual housing 6 is provided with the display screen 9 that is disposed towards the rear of the display housing 6. A viewing opening or window 8 in the upper side of the display housing 6 towards the front end of the display housing 6 allows a user to view a partially transparent reflective element 7 from a viewing area V. The partially transparent reflective element 7 is disposed in the lower side of the display housing 6 towards the front end of the display housing 6 and allows a user to see the workspace W from the viewing area V through the partially transparent reflective element 7. The partially transparent reflective element 7 is arranged to reflect an image displayed on the display screen 9 to the eyes of the user whilst the workspace W is simultaneously visible for the user through the partially transparent reflective element 7 (assuming that the eyes of the user are located in the viewing area V and the user is looking towards the partially transparent reflective element 7). Thus, in the view of the user, the virtual images of the virtual environment are mixed with images of the reality of the workspace W.

The display screen 9 and the partially transparent reflective element 7 are positioned such that the view is co-located with the position of the first handpiece 30. This allows the system to produce images of a virtual dental drill 30' that line up in the line of sight of the user with real-world first handpiece 30.

The dental procedure simulator is configured to reflect the images from the display screen 9 to the eyes of a user by reflection on the partially transparent reflective element 7 and is configured to mix the images of the virtual environment with a view of the workspace W seen by the user through the partially transparent reflective element 7.

Thus, the images on the display screen 9 are reflected to the eyes of the user, and the workspace W is simultaneously visible for the user through the partially transparent reflective element 7 when the user looks at the semi-reflective element 7 from the viewing space V.

In an embodiment, the display screen 9 is a stereoscopic display screen and the computer 80 is configured to send stereoscopic images to the stereoscopic display screen 9. In an embodiment, the stereoscopic display screen 9 is an autostereoscopic display screen 9. In an embodiment, the display screen 9 stereoscopic display screen in which the level of stereo is adjustable so that it can be adjusted to the optimal level for a particular user.

The computer 80 is configured to provide a three-dimensional virtual environment comprising a first virtual tool 30' having a first virtual position and a first virtual orientation, the first virtual tool 30' corresponding in size and shape to the handpiece 30 and the first virtual tool 30' being co-located with the handpiece 30.

The computer 80 sends images of the simulated dental procedure or treatment to the display screen 9, The images on the display screen are reflected to a user via a semi-transparent reflective element 7 (such as e.g. a semi-transparent mirror) to the eyes of a user (assuming that the eyes of a user are located in a vision space V and the user is facing the semi-transparent reflective element 7). The vision space V is a three dimensional-space where a user can simultaneously observe the images from the display screen 9 through reflection by the semi-transparent reflective element 7 and objects in the workspace W through the semi-transparent reflective element 7.

The software is configured to present a virtual environment that includes at least one virtual object, such as e.g. a virtual tooth, all of which are viewed by a user via the partially transparent reflective element 7. The virtual environment includes in this embodiment virtual tool, in this embodiment a virtual dental drill 30' corresponding to real-world haptic drill handle 30 together with a virtual end effector, i.e. a virtual burr 33.

Referring now particularly to Figs. 7 and 8, a phantom upper jaw 13 and the phantom lower jaw 14 are supported by the structure of the dental procedure simulator 1 and arranged in the workspace W. The phantom lower jaw 14 is arranged (manually) movable relative to the phantom upper jaw 13. The phantom lower jaw 14 is suspended from the phantom upper jaw 13 by a hinge mechanism 15, in an embodiment a four-bar linkage. Preferably, the hinge mechanism 15 imitates the movement of a human jaw to render the model realistic.

The phantom lower jaw 14 is suspended from the phantom upper jaw 13 to allow movement between a fully open position (shown in the figures) and a closed position (not shown), respectively. A position sensor (not shown) is configured to generate a signal indicative of the position of the phantom lower jaw 14 relative to the phantom upper jaw 13 and is in data connection with the computer 80. The software is configured to adjust the position of a virtual lower jaw to the signal of the position sensor.

The phantom upper jaw 13 is suspended from the support structure to allow rotation in three degrees of freedom, with the center of rotation for each degree of freedom being located between the phantom upper jaw 13 and the phantom lower jaw 14, i.e. in the center of the workspace W, so that the phantom upper jaw 13 does not leave the workspace W then it is rotated. Three rotary position sensors (not shown) for sensing rotation of the upper jaw 13 are provided for sensing rotation in each of the three degrees of freedom. The computer 80 receives a signal from the three rotary position sensors. The software is configured to adjust the simulation of the dental procedure or treatment to the signal from the rotary position sensors, in particular, the software adjusts the orientation and position of the virtual upper jaw and the orientation and position of the virtual upper jaw.

The phantom lower jaw 14 can manually be rotated relative to the phantom upper jaw 13, with the jaw angle between the upper jaw 13 and lower jaw 14 being limited not to exceed 55°, preferably not to exceed 50° and even more preferably not to exceed 45°, to reflect the limitations of maximum jaw opening of a real patient.

In an embodiment, the default orientation of the phantom head 10 corresponds to a position of a patient in a fully reclined dental chair, i.e. with the face of the patient being directed substantially upwards. The phantom head 10 can be manually rotated around three axes, to an extent that reflects the possibilities for a real patient to rotate their head relative to their body.

The phantom upper jaw 13 can be provided with a removable generic phantom upper jaw element (not shown), removably attached thereto, and the phantom lower jaw 14 can be provided with a removable generic phantom lower jaw element (not shown) removably attached thereto. The generic phantom jaw elements have in an embodiment no phantom teeth but instead, have a more generic form that roughly corresponds to the shape of a jaw with teeth.

In the shown embodiment the phantom upper jaw 13 is provided with a specific phantom upper jaw element 21, and the phantom lower jaw 14 is provided with a specific element lower jaw element 20. The specific phantom lower jaw element 20 and the specific phantom upper jaw element 21 are provided with phantom teeth 22. The phantom teeth 22 are removably attached by the phantom teeth 22 being inserted in a specific recess 23 in the specific phantom upper or lower jaw element 20,21. In an embodiment, the specific phantom lower and upper jaw elements 20,21 with their phantom teeth 22 are accurate models of a portion of a real human upper jaw with its upper teeth and lower jaw with its lower teeth. The phantom tooth or teeth 22 that is/are is to be subject of the dental procedure or treatment is/are removed to provide space for the first handpiece 30 to move unhindered by the phantom tooth or teeth 22 concerned. In Figs. 7 to 8 one phantom tooth 22 has been removed by way of example and the recess 23 in the phantom jaw concerned 20,21 is empty. The remaining phantom teeth 22 can be used by the user to support the user's hands and/or fingers. The virtual tooth that is to be worked on is a virtual tooth corresponding to the location of the recess 23. Thus, there is no risk of the first handpiece 30 abutting in the real world with a phantom tooth 22, since the position at which the virtual tooth is located is not provided with the phantom tooth 22.

When a specific phantom upper and/or lower jaw element 20,21 is used the computer 80 is provided with a virtual model of the specific phantom lower jaw element 20 and/or of the specific upper jaw element 21. The computer 80 instructs the user which jaw element (generic or specific) is to be installed for a given exercise. Thus, the computer 80 is configured to instruct a user to install a generic upper or lower jaw element (not shown) or a particular specific phantom upper or lower jaw element 20,21.

In the embodiment shown in Figs. 7 and 8 the phantom upper jaw 13 and phantom lower jaw 14 are part of a phantom head 10. The phantom head 10 with its phantom lower jaw 14 and phantom upper jaw 13 is arranged movably relative to the support structure of the dental procedure simulator 1 and the phantom head 10 with its phantom lower jaw 14 and phantom upper jaw 13 to move in unison with one another.

Fig. 9 illustrates how the tip 31 of the handpiece 30 protrudes between the phantom upper jaw 13 and the phantom lower jaw of 14 into the workspace W. Since the first handpiece 30 is suspended from its proximal end 39 to the haptic arm 40, it is not a problem to insert the distal end 31 into the cavity between the upper jaw 13 in the lower jaw 14 and there is no risk of any part of the haptic arm including the mechanism 100 abutting with the phantom upper jaw 13 or the defendant lower jaw 14.

Referring now particularly to Figs. 10 and 11, which illustrate the linkage 40 that is controlled by the computer 80 to simulate the medical procedure or treatment. The linkage 40 comprises a main link 41 (in an embodiment an elongated straight member) and the first handpiece 30 is connected to a front extremity of the main link 41 by a mechanical joint with at least two degrees of freedom that will be explained in greater detail further below. In the present embodiment, the front extremity of the main link 41 is the controlled end of the main link 41. The control end of the haptic arm is formed by the distal end 31 of the first handpiece 30.

The linkage 40 has a first crank 42 driven by a first rotary actuator 47, a second crank 44 driven by a second rotary actuator 48, and a third crank 46 driven by a third rotary actuator 49. The respective rotation axes of the first, second, and third cranks 42,44,46 can in an embodiment (not shown) be arranged orthogonally relative to one another.

The rotation axis of the first crank 42 extends substantially vertically. The first crank 42 is coupled directly to the main link 41 at a first position which is at or near the rear extremity of the main link 41 by a hinge with two degrees of freedom, such as e.g. a universal joint.

The second crank 44 is coupled to the main link 41 via a first horizontally extending connecting rod 43 and the third crank 46 is coupled to the main link 41 via a second vertically extending connecting rod 45. The first crank 42 is arranged to actuate the main link 41 in a first (horizontal) axial direction X. The second crank 44 is arranged to actuate the main link 41 in a second (horizontal) transverse direction Y, and the third crank 46 is arranged to actuate the main link 41 in a second (vertical) transverse direction Z.

The first connecting rod 43 is coupled to the main link 41 at a second axial position between the front extremity and the first position and the second connecting rod 45 is coupled to the main link 41 at a third axial position between the front extremity and the first position. In an embodiment, the second and third axial position substantially coincide.

The main link 41 comprises a three-dimensional force sensor (3DoF sensor) 50 for sensing forces applied by the user to the first handpiece 30 in three dimensions. The three-dimensional force sensor 50 is disposed between the front extreme position and the second and/or third axial position, and the three-dimensional force sensor 50 preferably is an integral part of the main link 41. The three-dimensional force sensor 50 is coupled to the computer 80.

The first, second and third cranks 42,44,46 are coupled (directly or to the rotary motor driving the respective crank) to respective first second and third rotary position sensors or encoders 26,27,28, which are in data connection with the computer 80. In the shown embodiment, the rotation axis of the second crank 44 and of the third crank 46 both extend horizontally and parallel. However, the rotation axis of the second crank 44 and of the third crank 46 main embodiment also extends horizontal and at an angle to one another, for example, a right angle.

The first, second and third cranks 42,44,46 are mounted on a reference 51 (e.g. a frame or base). The reference 51 is supported by the main housing 4 or by the support structure of the dental procedure simulator 1.

The linkage 40 connects to the handpiece 30 via a mechanism 100 to the reference 51 and the linkage 40 provides six independent degrees of freedom for the handpiece 30 relative to the reference 51. The arrangement of the linkage 40 results in a workspace W that a shaped as a cuboid with a horizontal top and bottom.

The mechanism 100 comprises a first parallel four-bar linkage 110 coupled to a second parallel four-bar linkage 120. The proximal end of the handpiece 39 is connected to a first arm 102 of a pair of parallel arms of the first four-bar linkage 110. A third arm 126 of the second pair of parallel arms of the second four-bar linkage 120 connects to the controlled end of the main link 41, via a revolute joint 154 (see Figs. 12 to 19).

As shown in Figs. 12 to 19, the mechanism 100 allows the handpiece 30 to pivot rotate about three orthogonal axes A1,A2,A3 that intersect at a common point CP in space near the distal end 31 of the first handpiece 30. The common point CP is arranged in the workspace W and has a fixed position relative to the connection position 59 so that said common point CP moves in unison with the connection position 59. The common point CP is located at a fixed distance D from said connection position 59. In an embodiment, the connection position 59 is at the controlled end of the haptic arm 40, which is in this embodiment the free end of the main link 41.

Referring now particularly to Figs. 18 and 19, which illustrate the mechanism 100 and first handpiece 30 in greater detail.

The first handpiece 30 has a proximal end 39 and a distal end 31. The distal end 31 has a head 32. The head 32 is shaped and sized as a drill head of a real dental handpiece. The drill head of a real dental handpiece is configured to receive and drive a real burr. The virtual burr 33 (Fig. 6) is co-located with the head 32 by the virtual environment software running on the computer 80.

A first revolute joint 54 connects the handpiece 30 to the first arm 102 and allows the handpiece 30 to rotate about a first axis A1 that coincides with a longitudinal axis of the handpiece 30.

The first revolute joint 54 connects the handpiece 32 to a first arm 102. The first arm 102 is part of the first parallel four-bar linkage 110. The first parallel four-bar linkage 110 comprises parallel first and second arms 102,106.

The upper/outer half of the first pair of parallel links 104 is in the present embodiment formed by two parallel transversely spaced tubes. The first arm 102 is shaped like a yoke to allow the first arm 102 to connect to both of the two parallel transversely spaced tubes of the upper/outer half of the first pair of parallel links 104. The links of the first pair of parallel links 104 are kinked so that they do not collide with the first handpiece 30 when the first handpiece 30 is oriented in a nearly horizontal position with the distal end 31 of the first handpiece 30 pointing towards the connection point 154, as shown in Fig. 14. The first arm being shaped like a yoke ensures that the kinked part of the upper/outer half of the first pair of parallel links 104 does not collide with the kinked part of the lower/inner half of the first pair of parallel links 104

The lower/inner half of the first pair of parallel links 104 is in the present embodiment formed by a single tube.

The first parallel four-bar linkage 110 is thus formed by the pair of parallel first and second arm 102, 106 together with the first pair of parallel links 104, all connected to one another by hinged joints.

The second parallel four-bar linkage 120 is formed by a pair of parallel third arm fourth arm 126,122 and by a second pair of parallel links 124, all connected to one another by hinged joints.

In the present embodiment, the fourth arm 122 is formed by two parallel tubes that are an extension of the upper/outer part of the first pair of parallel links 104. The second arm 106 is an extension of the lower/inner half of the second pair of parallel links 124. Thus, the first parallel four-bar linkage 110 and the second parallel four-bar linkage 120 are interconnected and allow the distal end 31 of 30 first handpiece to rotate about a second axis A2.

The lower/inner half of the second pair of parallel links 124, is like the lower half of the first pair of parallel links 104 formed by a single tube. The upper/outer half of the second pair of parallel links 124 is like the upper half of the first pair of parallel links 104 formed by two transversely spaced tubes. Accordingly, the arm 126 is formed like a yoke so that it can connect to both transversely spaced tubes of the upper/outer half of the second pair of parallel links 124.

A first bracing 119 connects the pair of transversely spaced tubes of the upper half of the first pair of parallel links 104 and second bracing 129 connects the pair of transversely spaced tubes of the upper/outer half of the second pair of parallel links 124.

The parallel links 104 and 204 are formed by a pair of transversely spaced tubes or rods on either the inner or on the outer side, or in both the inner and outer side, in order to increase the rigidity of the arm/mechanism 100. If this rigidity is not required, the parallel links 104 and 204 can be formed by a single tube, rod, or equivalent element.

A first transverse spacer 113 is arranged at the position where the upper/outer half of the first pair of parallel links 104 kinks.

The second arm 106 is T-shaped for acting as a transverse spacer and in a hinged connection with the tubes that form the upper/lower half of the first pair of parallel links 104 where these connect to the tubes that form the fourth arm 122.

A second transverse spacer 123 is arranged at the hinged connection between the tubes of the fourth arm 122 and the tubes that form the upper/outer half of the second pair of parallel links 124.

A third transverse spacer is arranged between the tubes that form the upper/outer half of the second pair of parallel links 124, at a position close to where these tubes are hinged to the third arm 126.

In the present embodiment, the third arm 126 forms a bracket 128 that connects to the main link 41 via a second revolute joint 154. The second revolute joint 154 allows the complete mechanism 100 to rotate about a third axis A3. In an embodiment, a second rotational movement sensor (not shown) is associated with the second revolute joint 154 and in data connection with the computer 80, to inform the computer 80 about rotation of the first handpiece 30 about the third axis A3.

The construction of the mechanism 100 allows the user to change the orientation of the first handpiece 30 by rotation of the distal end 31 about the first, second and third axis A1, A2, A3 without noticeable resistance. The effect of the weight of the mechanism 100 and the first handpiece 30 is in an embodiment offset/balanced by a compensation mechanism. Embodiment the compensation mechanism comprises counterweights and/or resilient elements.

The mechanism allows the first handpiece 30 to be connected at its proximate end 39 with the axes of rotation A1,A2,A3 of the first handpiece 30 arranged at or near the distal end 31 and with the connection position on the haptic arm 40 at a fixed distance D and relative position from the distal end 31.

The first, second, and third axes A1,A2,A3 intersect at a position in space that is fixed relative to the second revolute joint 154, and at a fixed distance D to the second revolute joint 154.

The mechanism 100 together with the first revolute joint 54 and the second revolute joint 154 add three degrees of freedom to the six degrees of freedom provided by the haptic arm 40, thereby providing the first handpiece 30 with six degrees of freedom (three translational degrees of freedom plus three rotational degrees of freedom).

Thus, the distal end 31 rotates about a common point CP. In an embodiment, the common point CP corresponds to the tip of a virtual burr 33 that is virtually connected to (co-located with) the distal end 31. In this case, all rotation is about the tip CP of the virtual burr 33. Accordingly, the user experiences the forces of the haptic force feedback as acting on the distal end 31 or at the tip of the virtual end effector, like they do in a real medical procedure (on the distal end of the real tool or on the distal end of the real end effector). Further, all translations are through planes that intersect with CP, so that it does not have a moment arm with records to CP, which ensures that the user only feels the appropriate translational and rotational forces.

For the simulation to be accurate, the mechanism 100 should be rigid, i.e. stiff so that it flexes little when force is applied to the first handpiece 30. Further, the mechanism 100 should be light so that the mass inertia of the mechanism 100 influences the simulation and the user experience as little as possible.

Thus, the mechanism 100 should be both light and stiff. Therefore, in an embodiment, the first pair of parallel links 104 and the second pair of parallel links 124 are preferably formed from hollow tubular members and made from lightweight material with a high Young's modulus, such as e.g. a fiber-reinforced material comprising carbon fiber fibers. The same construction material can be used for the fourth arm 122.

First arm 102, the second arm 106, the third arm 126, the first transverse spacer 113, the second transverse spacer 123 and the third traverse spacer 125, as well as the bracing 119 and 129 are preferably made from polymer material, possibly fiber-reinforced polymer material, or from a lightweight metal, such as e.g. aluminum. Both the polymer material and the lightweight metal version can be 3D printed products.

In an embodiment, an inertial measurement unit 52 is incorporated in the first handpiece 30.

In an embodiment a rotary position sensor (not shown) that senses rotational movement of the first handpiece 30 relative to the arm 102 about the first revolute joint 54.

The inertial measurement unit 52 and/or the rotary position sensor is in data connection with the computer 80, preferably by a data cable or alternatively, wirelessly, for transmission of position and/or orientation data.

The inertial measurement unit 52 is positioned within the handpiece 30 and is configured to measure translational acceleration, rotational velocities and the magnetic field.

As such, the first inertial measurement unit 52 is also capable of determining the speed and displacement of the first handpiece 30 using data processing techniques known in the art. In an embodiment, the first inertial measurement unit 52 has nine sensors, which comprise a 3-axis gyroscope, 3-axes accelerometer, and 3-axes magnetometer. The first inertial measurement unit 52 is provided with an embedded Digital Motion Processor that acquires data from accelerometers, gyroscopes, magnetometers and processes the data. The inertial measurement unit chip outputs a quaternion, which describes the orientation in space to a reference, e.g. in real space. This data output is passed along the cable 58 along with the signal from a fourth rotary position sensor (not shown) in the handpiece to the computer 80.

The inertial measurement unit 52 is calibrated before use by placing the handpiece 30 with a defined orientation so that the world reference is aligned.

In an embodiment, the computer 80 is configured to simulate a medical procedure or treatment through haptic feedback, preferably haptic force feedback, with the linkage 40 with its associated actuators 47, 48, 49 and through visual feedback with the display screen 9. Hereto, the computer 80 is configured to use the signal from the three-dimensional force sensor 50 as input and by controlling the position of the extremity of the linkage 40 accordingly.

In an embodiment, the computer 80 includes software applications for providing a training platform, providing instructional material and videos, recording, replaying, and evaluating a user's performance; providing audio, visual and textual communication with a remote instructor over a computer network; providing a remote instructor ability to provide force inputs to the haptic system; and providing differing virtual objects (e.g. teeth, jaws or complete heads), tools and physical rules into the virtual environment.

In an embodiment, the computer 80 is configured to detect collision between a virtual burr 33 of a virtual dental drill (using the real position of the first tool 30), to determine the interaction force to be applied to the virtual drill based upon virtual drill position, a virtual drill model and a virtual tooth model. The computer 80 is also configured to calculate the virtual drill speed based upon the interaction force and user input, such as from a foot pedal.

In an embodiment, the tooth model volume is represented as a set of three-dimensional pixels or voxels. Each voxel has a hardness value associated with it, representing the type/quality of tooth material (i.e. dentin, enamel, pulp). The conventional marching cubes algorithm is used to create a triangle mesh of an isosurface of the tooth model voxel set.

The virtual handpiece 30' is modeled analytically or by voxels. Thus, the handpiece's physical model a finite number of voxels, or by a complete analytically defined shape. The handpiece model also has a vector parameter for the handpiece's three-dimensional velocity. The virtual tool is provided with a virtual burr 33. The virtual burr 33 or virtual handpiece 30' can come in virtual contact with the virtual tooth. Hereto, the shape of the virtual burr is rendered against the voxels of the virtual tooth. The real position of the first handpiece 30 is used to determine the position of the virtual burr 33 and to determine contact between the virtual burr 33 and the virtual tooth.

Referring now in particular to Fig. 21 a control loop is used to control the velocity in one direction of the end of the main link 41 and thereby the velocity of the first handpiece 30. In total 3 of these force control loops are active to control the 3 directions of movement (3DOF). The force control loop uses the difference between the virtual force that is calculated by virtual environment 90 and the real force in one direction that is calculated from the force measured by the 3DoF (Degrees of Freedom) force sensor 50 at a summation point 86. The output of the summation point 86 is the input for a lead-lag compensator 87 that removes high frequencies and connects into a standard PI or PID controller 88. The PI or PID controller calculates a velocity command for the motor drive 89. The motor drive 89 is also in receipt of a signal from the rotary position sensors (encoders) 26, 27, 28 and determines the actual velocity of the handpiece 30 from the position signal. The motor drive 89 electrically drives the first rotary actuator 47, (the motor drives of the other two control loops drive the second rotary actuator 48 and the third rotary actuator 49). The motor drive uses the difference between the velocity command of the PI or PID controller and the real velocity that is calculated from the real position that is measured by the position sensor (encoder) 26,27,28 on the respective rotary actuator 46,47,48. A differentiator 92, that is in receipt of the position signal provides the actual velocity as output signal. The output of the differentiator 92 is provided to the motor drive 89 and to the virtual environment 90. In an embodiment, the differentiator 92 is an integral part of the motor drive 89. The first second and third rotary actuators 47, 48, 49 are connected to the first handpiece 30 via the mechanism 100 and the linkage 40 that connects to the various sensors (force, position, and orientation) described above. Input from a foot pedal sensor 91 is used to determine the rotary speed of the virtual burr 33. In an embodiment, the virtual environment receives a signal from IMU 52 to be informed of the orientation of the first handpiece 30. The virtual environment 90 includes a burr model, tooth model, and a jaw model and uses the real position and the real orientation of the first handpiece 30 to determine the position and orientation of the virtual burr 33. The virtual burr model and the virtual tooth model or jaw model are used to calculate the resulting virtual force that is sent back as a command to the force control loop and applied to the first handpiece 30.

When beginning a dental procedure simulator 1, the user positions herself in a chair (not shown) in front of the dental procedure simulator 1. If the display screen 9 is an autostereoscopic display screen the user does not need to use shutter glasses or glasses with polarized lenses, otherwise the user will put on shutter glasses or glasses with polarized lenses. The height of the main housing 4 is properly adjusted to the ideal working height for the user concerned. The chair height can also be adjusted according to the need of the user concerned.

The dental procedure simulator is in an embodiment provided with a network connection through the computer 80.

The computer 80 can be programmed to enhance the user experience by audio information through a loudspeaker. Thus, the training experience can be enhanced by providing instructions or feedback on user performance on the display screen 9 and via a loudspeaker.

The computer 80 has at least a first mode of operation for simulating a dental procedure or treatment using the handpiece 30 and a second mode of operation for training a dental procedure or treatment using the conventional powered dental handpiece 130.

In an embodiment, the first parallel four-bar linkage 110 and/or the second parallel four-bar linkage 120 are enveloped by a covering. The covering prevents a user from inadvertently getting their fingers pinched between the members of the first and second parallel four-bar mechanisms.

In this disclosure, any reference to a body part, such e.g. teeth, lower jaw, upper jaw, or head, typically referred to the human versions of these body parts. Thus, in this disclosure e.g. phantom lower jaw is a physical model of a human lower jaw and e.g. a virtual lower jaw is a virtual model of the human lower jaw.

The various aspects and implementations have been described in conjunction with various embodiments herein. However, other variations to the disclosed embodiments can be understood and effected by those skilled in the art in practicing the claimed subject-matter, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems.

The reference numerals used in the claims shall not be construed as limiting the scope. Unless otherwise indicated, the drawings are intended to be read (e.g., cross-hatching, arrangement of parts, proportion, degree, etc.) together with the specification, and are to be considered a portion of the entire written description of this disclosure. As used in the description, the terms "horizontal", "vertical", "left", "right", "up" and "down", as well as adjectival and adverbial derivatives thereof (e.g., "horizontally", "rightwardly", "upwardly", etc.), simply refer to the orientation of the illustrated structure as the particular drawing figure faces the reader. Similarly, the terms "inwardly" and "outwardly" generally refer to the orientation of a surface relative to its axis of elongation, or axis of rotation, as appropriate.

## Claims

1. A medical procedure simulator (1) for simulating a medical treatment or procedure, said medical procedure simulator (1) comprising:
a handpiece (30) with a proximal end (39) and a distal end (31),
said handpiece (30) being configured to be manipulated by a user in a workspace (W) in real space,
a haptic arm (40) controlled by a computer (80) that is configured to simulate a medical procedure or treatment through haptic feedback, preferably haptic force feedback, to said handpiece (30) with said haptic arm,
**characterized by** said medical procedure simulator (1) further comprising:
a mechanism (100) connected to said proximal end (39) of said handpiece (30) and connected to said haptic arm at a connection position (59) on said haptic arm,
said mechanism (100) being configured to allow said handpiece (30) to rotate about three orthogonal axes (A1,A2,A3) that intersect through said common point (CP) in said workspace (W),
said common point (CP) being a fixed position and distance relative to said connection position (59) so that said common point (CP) moves in unison with said connection position (59),
said mechanism comprising a first arm (102) that is connected to said proximal end (39) by a revolute joint (54) that allows said handpiece (30) to rotate about a first axis (A1) that coincides with the longitudinal axis of said handpiece (30),
said mechanism (100) comprising a first parallel four-bar linkage (110) operatively coupled to a second parallel four-bar linkage (120) to allow rotation of said handpiece (30) about a second axis (A2) coinciding with a common point (CP),and
said second parallel four-bar linkage being connected to said haptic arm (40) by a second revolute joint (154), said second revolute joint (154) allowing rotation about third axis (A3) parallel with the extent of said haptic arm, said third axis coinciding with said common point (CP).

2. A medical procedure simulator (1) according to claim 1, wherein said common point (CP) substantially coincides with said distal end (31).

3. The medical procedure simulator (1) according to claim 1 or 2, wherein said first arm (102) is part of a first pair of parallel arms of said first parallel four-bar linkage (110).

4. The medical procedure simulator (1) according to any one of claims 1 to 3, wherein said haptic arm comprises linkage (40) comprising a main link (41) that is operably coupled to a reference (51) by actuators (47,48,49), said main link (41) comprising a three-dimensional force sensor (50) for sensing forces applied by said user to said handpiece (30) in three dimensions, said three-dimensional force sensor (50) being disposed between said connection position (59) and any position at which said actuators (47,48,49) connect to said main link (41), and said three-dimensional force sensor (50) preferably being an integral part of said main link (41).

5. The dental procedure simulator (1) according to any one of claims 1 to 4, wherein said first revolute joint (54) is provided with a rotary position sensor (53) for sensing the rotary position of said handpiece (30) relative to said first arm (102).

6. The medical procedure simulator (1) according to any one of claims 1 to 5, wherein an inertial measurement unit (52) is arranged inside said handpiece (30).

7. The medical procedure simulator (1) according to any one of claims 1 to 6, comprising a phantom upper jaw (13) and a phantom lower jaw (14), preferably as a part of a phantom head (10), said phantom lower jaw (14) being arranged at a jaw angle to said phantom upper jaw (13), said jaw angle not exceeding 55°, preferably not exceeding 50° and even more preferably not exceeding 45°, and said connection position being located, inferior, preferably inferomedial to said lower jaw.

8. The medical procedure simulator (1) according to claim 7, wherein said lower jaw is arranged pivotable relative to said upper jaw between a closed position and an open position, the jaw angle at said open position not exceeding 55°, preferably not exceeding 50° and even more preferably not exceeding 45°.

9. A mechanism (100) for connecting a handpiece (30) of a medical simulator (1) to a haptic arm (40) of a medical simulator (1) according to any one of claims 1 to 8, said mechanism (100) comprising:
a first arm (102) with a first revolute joint (54) for connecting to said handpiece (30), said first revolute joint allowing having an axis of rotation allowing said handpiece (30) to rotate about a first axis (A1) that coincides with a longitudinal axis of said handpiece (30),
said first arm (102) being part of a first parallel four-bar linkage (110), said first parallel four-bar linkage (110) being operably connected to a second four-bar linkage (120) to allow said first arm (102) to rotate about a second axis (A2),
said second parallel four-bar linkage (120) comprising a third arm (126) with a second revolute joint (154) for connecting to said haptic arm (40) via, said second revolute joint (154) allowing said first arm (102) to rotate about a third axis (A3) that is preferably parallel with said haptic arm, and
said three orthogonal axes (A1,A2,A3) intersecting through said a common point (CP) in a workspace (W).

10. The mechanism (100) according to claim 9, wherein said first, second and third axis substantially coincide at a common point (CP) at or near said distal end (31).

11. The mechanism (100) according to claim 10, wherein said first, second and third axes intersect at a position in space that is fixed relative to said second revolute joint (154), and at a fixed distance to said second revolute joint (154).

12. The mechanism (100) according to any one of claims 9 to 11, wherein said first parallel four-bar linkage (110) comprises a first pair of parallel links (104) and a first pair of parallel arms,
wherein said second parallel four-bar linkage (120) comprises a second pair of parallel links (124) and a second pair of parallel arms,
wherein said first pair of parallel arms is formed by said first arm (102) and by an arm formed by an extension (106) of one link of said second pair of parallel links (124), and
wherein said second pair of parallel arms is formed by said third arm (126) and by an arm (122) formed by an extension of one link of said first pair of parallel links (124).

13. The mechanism (100) according to any one of claims 9 to 12, wherein one link of said first pair of links (104) and one link of said second pair of links (124) is formed by two parallel members that are spaced in the direction of said second axis, said two parallel members preferably being interconnected by a bracing (119,129).

14. The mechanism (100) according to any one of claims 9 to 13, wherein the links of said first pair of links (104) are kinked and/or wherein said first parallel four-bar linkage (110) and/or said second parallel four-bar linkage (120) are enveloped by a covering.

15. The mechanism (100) according to any one of claims 9 to 14, wherein said mechanism (100) is equipoised by one or more resilient members operably coupled to said mechanism (100), said resilient members preferably being long elastic cords or cables.

## Patentansprüche

1. Ein Simulator für medizinische Eingriffe (1) zum Simulieren einer medizinischen Behandlung oder eines medizinischen Eingriffs, wobei der Simulator für medizinische Eingriffe (1) umfasst:
ein Handstück (30) mit einem proximalen Ende (39) und einem distalen Ende (31),
wobei das Handstück (30) dazu konfiguriert ist, von einem Benutzer in einem Arbeitsraum (W) im realen Raum gehandhabt zu werden,
einen von einem Computer (80) gesteuerten haptischen Arm (40), der dazu konfiguriert ist, einen medizinischen Eingriff oder eine medizinische Behandlung durch haptische Rückmeldung, vorzugsweise haptische Kraftrückmeldung, an das Handstück (30) mit dem haptischen Arm zu simulieren,
**dadurch gekennzeichnet, dass** der Simulator für medizinische Eingriffe (1) ferner umfasst:
einen Mechanismus (100), der mit dem proximalen Ende (39) des Handstücks (30) verbunden ist und an einer Verbindungsposition (59) an dem haptischen Arm mit dem haptischen Arm verbunden ist,
wobei der Mechanismus (100) dazu konfiguriert ist, zuzulassen, dass das Handstück (30) um drei orthogonale Achsen (A1, A2, A3) rotiert, die sich in dem gemeinsamen Punkt (CP) in dem Arbeitsraum (W) schneiden,
wobei der gemeinsame Punkt (CP) eine feste Position und einen festen Abstand relativ zu der Verbindungsposition (59) aufweist, sodass sich der gemeinsame Punkt (CP) gemeinsam mit der Verbindungsposition (59) bewegt,
wobei der Mechanismus einen ersten Arm (102) umfasst, der mit dem proximalen Ende (39) durch ein Drehgelenk (54) verbunden ist, das zulässt, dass das Handstück (30) um eine erste Achse (A1) rotiert, die mit der Längsachse des Handstücks (30) zusammenfällt,
wobei der Mechanismus (100) ein erstes paralleles Vierstabgestänge (110) umfasst, das mit einem zweiten parallelen Vierstabgestänge (120) wirkgekoppelt ist, um eine Rotation des Handstücks (30) um eine zweite Achse (A2), die mit einem gemeinsamen Punkt (CP) zusammenfällt, zuzulassen, und
wobei das zweite parallele Vierstabgestänge mit dem haptischen Arm (40) durch ein zweites Drehgelenk (154) verbunden ist, wobei das zweite Drehgelenk (154) eine Rotation um eine dritte Achse (A3) parallel zu der Erstreckung des haptischen Arms zulässt, wobei die dritte Achse mit dem gemeinsamen Punkt (CP) zusammenfällt.

2. Simulator für medizinische Eingriffe (1) nach Anspruch 1, wobei der gemeinsame Punkt (CP) im Wesentlichen mit dem distalen Ende (31) zusammenfällt.

3. Simulator für medizinische Eingriffe (1) nach Anspruch 1 oder 2, wobei der erste Arm (102) Teil eines ersten Paars paralleler Arme des ersten parallelen Vierstabgestänges (110) ist.

4. Simulator für medizinische Eingriffe (1) nach einem der Ansprüche 1 bis 3, wobei der haptische Arm ein Gestänge (40) umfasst, das ein Hauptglied (41) umfasst, das durch Aktuatoren (47, 48, 49) mit einer Bezugsstruktur (51) wirkgekoppelt ist, wobei das Hauptglied (41) einen dreidimensionalen Kraftsensor (50) zum Erfassen von Kräften, die von dem Benutzer in drei Dimensionen auf das Handstück (30) ausgeübt werden, umfasst, wobei der dreidimensionale Kraftsensor (50) zwischen der Verbindungsposition (59) und einer beliebigen Position, an der die Aktuatoren (47, 48, 49) mit dem Hauptglied (41) verbunden sind, eingerichtet ist und wobei der dreidimensionale Kraftsensor (50) vorzugsweise ein integraler Bestandteil des Hauptglieds (41) ist.

5. Simulator für zahnärztliche Eingriffe (1) nach einem der Ansprüche 1 bis 4, wobei das erste Drehgelenk (54) mit einem Drehpositionssensor (53) zum Erfassen der Drehposition des Handstücks (30) relativ zu dem ersten Arm (102) bereitgestellt ist.

6. Simulator für medizinische Eingriffe (1) nach einem der Ansprüche 1 bis 5, wobei eine Trägheitsmesseinheit (52) innerhalb des Handstücks (30) angeordnet ist.

7. Simulator für medizinische Eingriffe (1) nach einem der Ansprüche 1 bis 6, umfassend einen Phantomoberkiefer (13) und einen Phantomunterkiefer (14), vorzugsweise als Teil eines Phantomkopfes (10), wobei der Phantomunterkiefer (14) in einem Kieferwinkel zu dem Phantomoberkiefer (13) angeordnet ist, wobei der Kieferwinkel 55° nicht überschreitet, vorzugsweise 50° nicht überschreitet und noch bevorzugter 45° nicht überschreitet, und wobei sich die Verbindungsposition inferior, vorzugsweise inferomedial, zu dem Unterkiefer befindet.

8. Simulator für medizinische Eingriffe (1) nach Anspruch 7, wobei der Unterkiefer relativ zu dem Oberkiefer zwischen einer geschlossenen Position und einer offenen Position schwenkbar angeordnet ist, wobei der Kieferwinkel in der offenen Position 55° nicht überschreitet, vorzugsweise 50° nicht überschreitet und noch bevorzugter 45° nicht überschreitet.

9. Ein Mechanismus (100) zum Verbinden eines Handstücks (30) eines medizinischen Simulators (1) mit einem haptischen Arm (40) eines medizinischen Simulators (1) nach einem der Ansprüche 1 bis 8, der Mechanismus (100) umfassend:
einen ersten Arm (102) mit einem ersten Drehgelenk (54) zum Verbinden mit dem Handstück (30),
wobei das erste Drehgelenk es zulässt, dass eine Rotationsachse vorhanden ist, die zulässt, dass das Handstück (30) um eine erste Achse (A1) rotiert, die mit einer Längsachse des Handstücks (30) zusammenfällt,
wobei der erste Arm (102) Teil eines ersten parallelen Vierstabgestänges (110) ist,
wobei das erste parallele Vierstabgestänge (110) mit einem zweiten Vierstabgestänge (120) wirkverbunden ist, um zuzulassen, dass der erste Arm (102) um eine zweite Achse (A2) rotiert,
wobei das zweite parallele Vierstabgestänge (120) einen dritten Arm (126) mit einem zweiten Drehgelenk (154) zum Verbinden mit dem haptischen Arm (40) über das zweite Drehgelenk (154) umfasst, wobei das zweite Drehgelenk (154) zulässt, dass der erste Arm (102) um eine dritte Achse (A3) rotiert, die vorzugsweise parallel zu dem haptischen Arm ist, und
wobei sich die drei orthogonalen Achsen (A1, A2, A3) in einem gemeinsamen Punkt (CP) in einem Arbeitsraum (W) schneiden.

10. Mechanismus (100) nach Anspruch 9, wobei die erste, die zweite und die dritte Achse im Wesentlichen in einem gemeinsamen Punkt (CP) an oder nahe dem distalen Ende (31) zusammenfallen.

11. Mechanismus (100) nach Anspruch 10, wobei sich die erste, die zweite und die dritte Achse an einer Position im Raum schneiden, die relativ zu dem zweiten Drehgelenk (154) fest ist und einen festen Abstand zu dem zweiten Drehgelenk (154) aufweist.

12. Mechanismus (100) nach einem der Ansprüche 9 bis 11, wobei das erste parallele Vierstabgestänge (110) ein erstes Paar paralleler Glieder (104) und ein erstes Paar paralleler Arme umfasst,
wobei das zweite parallele Vierstabgestänge (120) ein zweites Paar paralleler Glieder (124) und ein zweites Paar paralleler Arme umfasst,
wobei das erste Paar paralleler Arme durch den ersten Arm (102) und durch einen Arm gebildet wird, der durch eine Verlängerung (106) eines Glieds des zweiten Paars paralleler Glieder (124) gebildet wird, und
wobei das zweite Paar paralleler Arme durch den dritten Arm (126) und durch einen Arm (122) gebildet wird, der durch eine Verlängerung eines Glieds des ersten Paars paralleler Glieder (124) gebildet wird.

13. Mechanismus (100) nach einem der Ansprüche 9 bis 12, wobei ein Glied des ersten Paars von Gliedern (104) und ein Glied des zweiten Paars von Gliedern (124) durch zwei parallele Elemente gebildet werden, die in Richtung der zweiten Achse voneinander beabstandet sind, wobei die zwei parallelen Elemente vorzugsweise durch eine Verstrebung (119, 129) miteinander verbunden sind.

14. Mechanismus (100) nach einem der Ansprüche 9 bis 13, wobei die Glieder des ersten Paars von Gliedern (104) geknickt sind und/oder wobei das erste parallele Vierstabgestänge (110) und/oder das zweite parallele Vierstabgestänge (120) von einer Abdeckung umhüllt sind.

15. Mechanismus (100) nach einem der Ansprüche 9 bis 14, wobei der Mechanismus (100) durch ein oder mehrere elastische Elemente, die mit dem Mechanismus (100) wirkgekoppelt sind, im Gleichgewicht gehalten wird, wobei die elastischen Elemente vorzugsweise lange elastische Stränge oder Kabel sind.

## Revendications

1. Simulateur (1) d'intervention médicale destiné à simuler un traitement médical ou une intervention médicale, ledit simulateur (1) d'intervention médicale comprenant :
une pièce à main (30) avec une extrémité proximale (39) et une extrémité distale (31),
ladite pièce à main (30) étant configurée pour être manipulée par un utilisateur dans un espace de travail (W) dans l'espace réel,
un bras haptique (40) commandé par un ordinateur (80) qui est configuré pour simuler une intervention médicale ou un traitement médical par le biais d'un retour haptique, de préférence un retour de force haptique, vers ladite pièce à main (30) à l'aide dudit bras haptique,
ledit simulateur (1) d'intervention médicale étant **caractérisé en ce qu'**il comprend en outre :
un mécanisme (100) relié à ladite extrémité proximale (39) de ladite pièce à main (30) et relié audit bras haptique au niveau d'une position de connexion (59) sur ledit bras haptique,
ledit mécanisme (100) étant configuré pour permettre à ladite pièce à main (30) de tourner autour de trois axes orthogonaux (A1, A2, A3) qui se coupent en un point commun (CP) dans ledit espace de travail (W),
ledit point commun (CP) étant une position et une distance fixes par rapport à ladite position de connexion (59), de sorte que ledit point commun (CP) se déplace à l'unisson avec ladite position de connexion (59),
ledit mécanisme comprenant un premier bras (102) qui est relié à ladite extrémité proximale (39) par une articulation rotoïde (54) qui permet à ladite pièce à main (30) de tourner autour d'un premier axe (A1) qui coïncide avec l'axe longitudinal de ladite pièce à main (30),
ledit mécanisme (100) comprenant une première liaison (110) à quatre barres parallèles couplée fonctionnellement à une seconde liaison (120) à quatre barres parallèles pour permettre la rotation de ladite pièce à main (30) autour d'un deuxième axe (A2) coïncidant avec un point commun (CP), et
ladite seconde liaison à quatre barres parallèles étant reliée audit bras haptique (40) par une seconde articulation rotoïde (154), ladite seconde articulation rotoïde (154) permettant une rotation autour d'un troisième axe (A3) parallèle au prolongement dudit bras haptique, ledit troisième axe coïncidant avec ledit point commun (CP).

2. Simulateur (1) d'intervention médicale selon la revendication 1, dans lequel ledit point commun (CP) coïncide sensiblement avec ladite extrémité distale (31).

3. Simulateur (1) d'intervention médicale selon la revendication 1 ou 2, dans lequel ledit premier bras (102) fait partie d'une première paire de bras parallèles de ladite première liaison (110) à quatre barres parallèles.

4. Simulateur (1) d'intervention médicale selon l'une quelconque des revendications 1 à 3, dans lequel ledit bras haptique comprend une liaison (40) comprenant une liaison principale (41) qui est couplée fonctionnellement à une référence (51) par des actionneurs (47, 48, 49), ladite liaison principale (41) comprenant un capteur (50) de force tridimensionnel destiné à détecter les forces appliquées par ledit utilisateur sur ladite pièce à main (30) dans les trois dimensions, ledit capteur (50) de force tridimensionnel étant disposé entre ladite position de connexion (59) et toute position à laquelle lesdits actionneurs (47, 48, 49) sont reliés à ladite liaison principale (41), et ledit capteur (50) de force tridimensionnel faisant de préférence partie intégrante de ladite liaison principale (41).

5. Simulateur (1) d'intervention dentaire selon l'une quelconque des revendications 1 à 4, dans lequel ladite première articulation rotoïde (54) est pourvue d'un capteur (53) de position rotatif destiné à détecter la position en rotation de ladite pièce à main (30) par rapport audit premier bras (102).

6. Simulateur (1) d'intervention médicale selon l'une quelconque des revendications 1 à 5, dans lequel une unité (52) de mesure inertielle est agencée à l'intérieur de ladite pièce à main (30).

7. Simulateur (1) d'intervention médicale selon l'une quelconque des revendications 1 à 6, comprenant une mâchoire supérieure fantôme (13) et une mâchoire inférieure fantôme (14), de préférence en tant que partie d'une tête fantôme (10), ladite mâchoire inférieure fantôme (14) étant agencée selon un angle de mâchoire par rapport à ladite mâchoire supérieure fantôme (13), ledit angle de mâchoire ne dépassant pas 55°, de préférence ne dépassant pas 50° et, plus préférablement encore, ne dépassant pas 45°, et ladite position de connexion étant située en position inférieure, de préférence en position inféro-médiale, par rapport à ladite mâchoire inférieure.

8. Simulateur (1) d'intervention médicale selon la revendication 7, dans lequel ladite mâchoire inférieure est agencée de manière à pouvoir pivoter par rapport à ladite mâchoire supérieure entre une position fermée et une position ouverte, l'angle de mâchoire dans ladite position ouverte ne dépassant pas 55°, de préférence ne dépassant pas 50° et, plus préférablement encore, ne dépassant pas 45°.

9. Mécanisme (100) servant à relier une pièce à main (30) d'un simulateur médical (1) à un bras haptique (40) d'un simulateur médical (1) selon l'une quelconque des revendications 1 à 8, ledit mécanisme (100) comprenant :
un premier bras (102) comportant une première articulation rotoïde (54) destinée à être reliée à ladite pièce à main (30), ladite première articulation rotoïde présentant un axe de rotation permettant à ladite pièce à main (30) de tourner autour d'un premier axe (A1) qui coïncide avec un axe longitudinal de ladite pièce à main (30),
ledit premier bras (102) faisant partie d'une première liaison (110) à quatre barres parallèles, ladite première liaison (110) à quatre barres parallèles étant reliée fonctionnellement à une seconde liaison (120) à quatre barres parallèles pour permettre audit premier bras (102) de tourner autour d'un deuxième axe (A2),
ladite seconde liaison (120) à quatre barres parallèles comprenant un troisième bras (126) muni d'une seconde articulation rotoïde (154) destinée à être reliée audit bras haptique (40) par l'intermédiaire de ladite seconde articulation rotoïde (154) permettant audit premier bras (102) de tourner autour d'un troisième axe (A3) qui est de préférence parallèle audit bras haptique, et
lesdits trois axes orthogonaux (A1, A2, A3) se coupant en un point commun (CP) dans un espace de travail (W).

10. Mécanisme (100) selon la revendication 9, dans lequel lesdits premier, deuxième et troisième axes coïncident sensiblement en un point commun (CP) situé au niveau ou à proximité de ladite extrémité distale (31).

11. Mécanisme (100) selon la revendication 10, dans lequel lesdits premier, deuxième et troisième axes se coupent en une position dans l'espace qui est fixe par rapport à ladite seconde articulation rotoïde (154), et à une distance fixe de ladite seconde articulation rotoïde (154).

12. Mécanisme (100) selon l'une quelconque des revendications 9 à 11, dans lequel ladite première liaison (110) à quatre barres parallèles comprend une première paire (104) de liaisons parallèles et une première paire de bras parallèles,
dans lequel ladite seconde liaison (120) à quatre barres parallèles comprend une seconde paire (124) de liaisons parallèles et une seconde paire de bras parallèles,
dans lequel ladite première paire de bras parallèles est formée par ledit premier bras (102) et par un bras formé par un prolongement (106) d'une liaison de ladite seconde paire (124) de liaisons parallèles, et
dans lequel ladite seconde paire de bras parallèles est formée par ledit troisième bras (126) et par un bras (122) formé par un prolongement d'une liaison de ladite première paire (124) de liaisons parallèles.

13. Mécanisme (100) selon l'une quelconque des revendications 9 à 12, dans lequel une liaison de ladite première paire (104) de liaisons et une liaison de ladite seconde paire (124) de liaisons sont formées par deux organes parallèles qui sont espacés dans la direction dudit deuxième axe, lesdits deux organes parallèles étant de préférence interconnectés par un renfort (119, 129).

14. Mécanisme (100) selon l'une quelconque des revendications 9 à 13, dans lequel les liaisons de ladite première paire (104) de liaisons sont coudées et/ou dans lequel ladite première liaison (110) à quatre barres parallèles et/ou ladite seconde liaison (120) à quatre barres parallèles sont enveloppées par un revêtement.

15. Mécanisme (100) selon l'une quelconque des revendications 9 à 14, ledit mécanisme (100) étant équilibré par un ou plusieurs organes élastiques couplés fonctionnellement audit mécanisme (100), lesdits organes élastiques étant de préférence de longs cordons ou câbles élastiques.
